# EUROPEAN PATENT APPLICATION

(11) **EP 4 442 307 A1**
(43) Date of publication of application: **09.10.2024**
(21) Application number: 22910936.8
(22) Date of filing: 08.12.2022
(51) Int. Cl.: A61M 31/00, A61B 17/00

(54) **MEDICAL DEVICE AND METHOD FOR APPLYING SHEET-FORM DELIVERY OBJECT**

(30) Priority: 22.12.2021 JP 2021208085
(71) Applicant: Terumo Kabushiki Kaisha, Tokyo 151-0072 (JP)
(72) Inventor: HAYAKAWA, Koichi, Ashigarakami-gun, Kanagawa 259-0151 (JP)
(74) Representative: Prüfer & Partner mbB Patentanwälte · Rechtsanwälte
(86) International application number: PCT/JP2022/045365
(87) International publication number: WO 2023/120225

(57) **Abstract**

A medical device (M) for applying a sheet-shaped delivery object (S) to a target site (B), the medical device (M) including a main body (1) having a long shaft portion (11) and a holding portion (12) provided at a distal end of the shaft portion (11) for holding the sheet-shaped delivery object (S), in which the holding portion (12) has a tapered portion (121) widening in a tapered manner from the distal end of the shaft portion (11) and a placement portion (122) extending from the tapered portion (121), and both end portions of the placement portion (122) are folded back inward at least on a proximal end side.

## Description

### Technical Field

The present invention relates to a medical device and method for applying a sheet-shaped delivery object.

### Background Art

In recent years, attempts have been made to transplant various cells for repair of damaged tissues and the like. For example, attempts have been made to use fetal cardiomyocytes, myoblast cells, mesenchymal stem cells, cardiac stem cells, ES cells, iPS cells, and the like for repair of myocardial tissue damaged by ischemic heart disease such as angina pectoris and myocardial infarction (Haraguchi et al., Stem Cells Transl Med. 2012 Feb; 1 (2) : 136-41) .

As a part of such attempts, a cell structure formed using a scaffold and a sheet-shaped cell culture in which cells are formed in a sheet shape have been developed (Sawa et al., Surg Today. 2012 Jan;42(2) :181-4).

For the application of the sheet-shaped cell culture to treatment, studies have been made on the use of a cultured epidermal sheet for skin damage caused by burns or the like, the use of a corneal epithelial sheet-shaped cell culture for corneal damage, the use of an oral mucosa sheet-shaped cell culture for endoscopic resection of esophageal cancer, and the like, and some of them have entered the stage of clinical application.

With regard to a surgical method for administering a sheet-shaped cell culture to a target site, endoscopic surgery is widely used as a minimally invasive surgical method for a human body, and various instruments for delivering and administering a sheet-shaped cell culture to a target site have been proposed.

For example, a conveyance and administration device described in JP 2009-511 A can convey a sheet-shaped therapeutic substance to an implantation site with a low degree of invasion to the human body by housing a sheet support member in a cylindrical shape in an outer cylinder.

A sheet pasting device described in JP 2016-187601 A can peel off a rod-like member disposed along a surface of a sheet support portion by moving the rod-like member along the surface of the sheet support portion to paste a sheet-like substance to a target site.

In a transport administration device described in JP 2008-173333 A, a sheet can be administered to a sheet support by a sheet attaching and detaching unit capable of applying a negative pressure for adsorbing and holding a sheet-like therapeutic substance and a positive pressure for detaching the sheet-like therapeutic substance from the sheet support.

A transfer device described in JP 2012-30045 A includes a displacement body provided to be displaceable with respect to a main body portion, and a belt body wound so as to cover the displacement body and connected to the main body portion, and can transfer a biological graft placed on the belt body by displacing the displacement body.

### Summary of Invention

The present inventors have faced a problem that it is difficult to efficiently apply the sheet-shaped delivery object to a target site while maintaining the shape of the sheet-shaped delivery object as described above, and the like. Therefore, it is an object of the present invention to solve such a problem and to realize a medical device for application to a target site while maintaining the shape of a sheet-shaped delivery object by a simple mechanism and a simple operation.

In making intensive studies in order to solve the above problem, the present inventors have found for the first time that a sheet-shaped delivery object can be efficiently applied to a target site while maintaining the shape of the sheet-shaped delivery object by adopting a simple mechanism. As a result of further continuing studies based on such findings, the present inventors have completed the present invention.

That is, the present invention relates to the following.
[1] A medical device for applying a sheet-shaped delivery object to a target site, the medical device including a main body having a long shaft portion and a holding portion provided at a distal end of the shaft portion for holding the sheet-shaped delivery object, and a long sliding body that slides on the holding portion, in which the sheet-shaped delivery object on the holding portion can be slidably moved to the target site by sliding the sheet-shaped delivery object while being pressed by the sliding body.
[2] The medical device according to [1], in which the holding portion is configured to be bendable at an arbitrary angle with respect to an axial direction of the shaft portion.
[3] The medical device according to [1] or [2], in which at least a part of the holding portion is curved along a circumferential direction of the main body.
[4] The medical device according to [1] or [2], in which at least a part of the holding portion is configured as a planar surface.
[5] The medical device according to any one of [1] to [4], in which the sliding body has a three-dimensional shaped or planar shaped pressing portion for pressing the sheet-shaped delivery object.
[6] The medical device according to any one of [1] to [5], in which the shaft portion is configured to be hollow.
[7] The medical device according to any one of [1] to [6], in which the holding portion has a tapered portion widening in a tapered manner from the distal end of the shaft portion and a placement portion extending from the tapered portion, and both end portions of the placement portion are folded back inward at least on a proximal end side.
[8] A medical device for applying a sheet-shaped delivery object to a target site, the medical device including a main body having a long shaft portion and a holding portion provided at a distal end of the shaft portion for holding a sheet-shaped delivery object, in which the shaft portion is configured to be hollow.
[9] The medical device according to [8], further including a long sliding body that slides on the holding portion in parallel with the main body.
[10] The medical device according to [5], in which the pressing portion has a pressing main body for pressing the sheet-shaped delivery object, and a grip portion protruding in an arch shape from the pressing main body.
[11] A method for applying a sheet-shaped delivery object to a target site, the method including the steps of providing the medical device of any one of [1] or [10], placing the sheet-shaped delivery object on the holding portion, delivering the medical device to a target site, and slidably moving the sheet-shaped delivery object on the holding portion by a sliding body.
[12] The method according to [11], in which the delivery to the target site is performed by inserting the medical device into a cylindrical body intraperitoneally inserted into a body cavity.
[13] The method according to [11] or [12], in which the sheet-shaped delivery object is intraperitoneally inserted into a body cavity and applied to the target site.
[14] A medical device for applying a sheet-shaped delivery object to a target site, the medical device including a main body having a long shaft portion and a holding portion provided at a distal end of the shaft portion for holding the sheet-shaped delivery object, in which the holding portion has a tapered portion widening in a tapered manner from the distal end of the shaft portion and a placement portion extending from the tapered portion, and both end portions of the placement portion are folded back inward at least on a proximal end side.
[15] The medical device according to [14], in which a folding angle of both end portions with respect to the placement portion is configured to increase from the distal end side to the proximal end side.
[16] The medical device according to [14] or [15], in which at least the distal end side of the placement portion on which the sheet-shaped delivery object is placed is configured to be a planar surface.
[17] The medical device according to any one of [14] to [16], in which the folding angle of both end portions with respect to the placement portion is configured to be 60 to 180 degrees, preferably 90 to 180 degrees.
[18] The medical device according to any one of [14] to [17], in which the holding portion is configured to be bendable at an arbitrary angle with respect to an axial direction of the shaft portion.
[19] The medical device according to any one of [14] to [18], in which the shaft portion is configured to be hollow.
[20] The medical device according to any one of [14] to [19], further including a long sliding body that slides on the holding portion and/or a cylindrical body.
[21] A medical device for applying a sheet-shaped delivery object to a target site, the medical device including a main body having a long shaft portion and a holding portion provided at a distal end of the shaft portion for holding the sheet-shaped delivery object, in which the holding portion includes a tapered portion widening in a tapered manner from a distal end of the shaft portion and a placement portion extending from the tapered portion, and when the main body is accommodated in a cylindrical body that can accommodate the main body so as to be movable forward and backward, both end portions of the placement portion are configured to be wound such that back surfaces thereof are pressed against each other.
[22] The medical device according to [21], in which the shaft portion is configured to be hollow.
[23] A method for applying a sheet-shaped delivery object to a target site, the method including the steps of providing the medical device of any one of [14] to [22], placing the sheet-shaped delivery object on a support portion, accommodating the medical device in a cylindrical body, delivering the medical device to a target site, and applying the sheet-shaped delivery object to the target site.
[24] The method according to [23], in which the delivery to the target site is performed by inserting the medical device into a cylindrical body intraperitoneally inserted into a body cavity.
[25] The method according to [23] or [24], in which the sheet-shaped delivery object is intraperitoneally inserted into a body cavity and applied to the target site.

The present invention can efficiently apply the sheet-shaped delivery object to a target site while maintaining the shape of the sheet-shaped delivery object by a simple mechanism and a simple operation, and therefore has a large advantage in terms of operability and manufacturing cost.

### Brief Description of Drawings

Fig. 1A is a conceptual diagram illustrating a medical device according to a first embodiment of the present invention. Fig. 1B is a diagram illustrating a configuration in which a sliding body is included in the medical device of Fig. 1A.
Fig. 2 is a diagram illustrating a usage example of the medical device according to the first embodiment of the present invention.
Fig. 3 is a conceptual diagram illustrating a medical device according to a second embodiment of the present invention.
Fig. 4 is a diagram illustrating a usage example of the medical device according to the second embodiment of the present invention.
Fig. 5 is a conceptual diagram illustrating a medical device according to a third embodiment of the present invention.
Fig. 6A is a diagram illustrating a usage example of the medical device according to the third embodiment of the present invention. Fig. 6B is a cross-sectional view taken along line VIB-VIB of Fig. 6A. Fig. 6C is a cross-sectional view taken along line VIC-VIC in Fig. 6A.
Fig. 7A is a view illustrating a modification of the medical device according to the third embodiment of the present invention. Fig. 7B is a view illustrating a configuration in which a sliding body is included in the medical device according to the third embodiment of the present invention. Fig. 7C is a view illustrating a first modification of the sliding body of Fig. 7B. Fig. 7D is a view illustrating a second modification of the sliding body in Fig. 7B. Fig. 7E is a view illustrating a third modification of the sliding body in Fig. 7B.
Fig. 8A is a view illustrating a fourth modification of the medical device according to the third embodiment of the present invention. Fig. 8B is a perspective view of a distal end portion of the sliding body shown in Fig. 8A. Fig. 8C is a front view of the sliding body of Fig. 8B as viewed from a distal end direction.
Fig. 9A is a first conceptual explanatory diagram of a folded portion according to the third embodiment of the present invention. Fig. 9B is a second conceptual explanatory diagram of the folded portion according to the third embodiment of the present invention. Fig. 9C is a third conceptual explanatory diagram of the folded portion according to the third embodiment of the present invention.
Fig. 10 is a cross-sectional view in a case where an endoscope is used for the medical device of the present invention.
Fig. 11A is a conceptual diagram illustrating another modification of the medical device of the present invention. Fig. 11 B is a conceptual diagram illustrating still another modification of the medical device of the present invention. Fig. 11C is a conceptual diagram illustrating still another modification of the medical device of the present invention.

### Description of Embodiments

In the present invention, the "sheet-shaped delivery object" means a sheet-shaped pharmaceutical product, a regenerative medicine product, a medical device, or the like that is delivered to a living body and used for treatment or the like. The sheet shape includes a film shape and a membrane shape (high viscosity object). The sheet-shaped pharmaceutical product includes a tissue adhesive, a local anesthetic, and the like. The sheet-shaped regenerative medicine product includes a graft, a cell culture, and the like. The sheet-shaped medical device includes an adhesion prevention material, a hemostatic material, a wound covering material, and the like.

In the present invention, the "graft" means a structure for transplantation into a living body, and particularly means a structure for transplantation containing living cells as a component. Examples of the graft of the present invention include, but are not limited to, a sheet-shaped cell culture, a spheroid, and a cell aggregate. A sheet-shaped cell culture or a spheroid is preferable, and a sheet-shaped cell culture is more preferable.

In the present invention, the "sheet-shaped cell culture" refers to a sheet-shaped culture in which cells are linked to each other. Cells may be linked to each other directly (including via a cellular element such as an adhesion molecule) and/or via an intervening substance. The intervening substance is not particularly limited as long as it is a substance capable of at least physically (mechanically) linking cells to each other, and examples thereof include an extracellular matrix. The intervening substance is preferably derived from a cell, in particular, derived from a cell constituting a cell culture. The cells are linked at least physically (mechanically), but may be further linked functionally, for example chemically or electrically. The sheet-shaped cell culture may be composed of one cell layer (monolayer) or composed of two or more cell layers (laminated (multilayer) body, for example, two layers, three layers, four layers, five layers, or six layers). In addition, the sheet-shaped cell culture may have a three-dimensional structure having a thickness exceeding the thickness of one cell without the cells exhibiting a clear layer structure. For example, in a vertical cross-section of the sheet-shaped cell culture, cells may be present in a state of being non-uniformly arranged (for example, in a mosaic manner) without being uniformly aligned in the horizontal direction.

The sheet-shaped cell culture preferably does not contain a scaffold (support). The scaffold may be used in the technical field in order to attach cells on and/or within a surface thereof and to maintain a physical integrity of the sheet-shaped cell culture. The sheet-shaped cell culture of the present invention can maintain physical integrity thereof without such a scaffold. The sheet-shaped cell culture of the present invention can also reinforce its physical strength by applying fibrin or the like.

The cells constituting the sheet-shaped cell culture are not particularly limited as long as they can form the sheet-shaped cell culture, and examples thereof include adherent cells (attaching cells). The adherent cells include, for example, adherent somatic cells, and the like. Examples of somatic cells include myoblasts (for example, myoblast cells), muscle satellite cells, mesenchymal stem cells (for example, those derived from bone marrow, adipose tissue, peripheral blood, skin, hair roots, muscle tissue, endometrium, placenta, and cord blood), cardiomyocytes, fibroblast cells, tissue stem cells such as cardiac stem cells, embryonic stem cells, pluripotent stem cells such as induced pluripotent stem (iPS) cells, synoviocytes, chondrocytes, epithelial cells (for example, oral mucosal epithelial cells, retinal pigment epithelial cells, and nasal mucosal epithelial cells), endothelial cells (for example, vascular endothelial cells), hepatocytes (for example, hepatocytes), pancreatic cells (for example, islet cells), renal cells, adrenal cells, periodontal ligament cells, gingival cells, periosteal cells, and skin cells. The somatic cells may be cells differentiated from iPS cells (iPS cell-derived cells), and examples thereof include iPS cell-derived cardiomyocytes, fibroblast cells, epithelial cells, endothelial cells, hepatocytes, pancreatic cells, renal cells, adrenal cells, periodontal ligament cells, gingival cells, periosteum cells, skin cells, synovium cells, and chondrocytes. The cells are preferably nucleated cells having a nucleus in the cell.

In the present invention, the "medical device" refers to a machine or instrument intended to be used for diagnosis, treatment, or prevention of diseases in humans or animals, or to affect the structure or function of the human or animal body.

In the present invention, the "target site" refers to a portion to which the sheet-shaped delivery object is applied (attached). Examples of the target site include a surface of the organ, a damaged site of the organ, an anastomosis site of the organ, and the like. The target site is typically a heart, a surface of the heart, a damaged site of the heart, an anastomosis site of the heart, or the like. In addition, examples of the target site include a site where a damage (wound) exists in a hollow organ, for example, a damaged site on the inner side of a lumen wall, an opposite side of the lumen wall corresponding to a site where a damage exists, and the like. The "hollow organ" means an organ having a lumen stored in a body cavity, that is, an organ having a tubular or bag-like structure, and examples thereof include organs of a digestive tract system, a circulatory system, a urinary tract system, a respiratory system, and a reproductive system.

Examples of the organ of the digestive tract system include esophagus, stomach, duodenum, pancreas, gallbladder, bile duct, small intestine, large intestine, and rectum. Among them, the duodenum is most preferable because the lumen wall is thin, the risk of perforation occurrence is high, and deterioration of damage easily progresses due to a severe environment exposed to various digestive fluids.

In one aspect, the sheet-shaped delivery object is immersed in a protective liquid. The protective liquid is not particularly limited as long as it is a liquid capable of maintaining the shape and function of the graft, and is, for example, a liquid such as physiological saline, phosphate buffer solution (PBS), Hank's balanced salt solution, cell culture solution, or water, or a mixture thereof.

In the present invention, the phrase "applying a sheet-shaped delivery object to a target site" refers to placing a sheet-shaped delivery object on a holding portion of a main body, delivering the main body on which the sheet-shaped delivery object is placed to a target site, moving the sheet-shaped delivery object on the holding portion from the proximal end side to the distal end side (using tweezers or a sliding body), and the like, in order to apply the sheet-shaped delivery object to the target site. The proximal end side of the main body is a side of an operator who operates the medical device, and the distal end side of the main body is a side opposite to the proximal end side and closer to the target site.

In the present invention, the "main body" has a long shaft portion and a holding portion provided at a distal end of the shaft portion for holding the sheet-shaped delivery object. The "shaft portion" has a rigidity and length to bring the sheet-shaped delivery object close to the target site while appropriately supporting the holding portion. The shaft portion can be configured to be solid or hollow (cylindrical shape, substantially tubular shape, or the like), and when the shaft portion is configured to be hollow, another long member (a sliding body, an endoscope, or the like) can be accommodated in the shaft portion so as to be movable forward and backward.

The "holding portion" has a surface capable of holding the sheet-shaped delivery object while maintaining its shape. The holding portion is preferably made of a flexible material. In this case, by curving the holding portion in a state of holding the sheet-shaped delivery object and storing the sheet-shaped delivery object along the inner side or the like of the cylindrical body, it is possible to deliver the sheet-shaped delivery object with a width larger than the inner diameter of the cylindrical body while protecting the sheet-shaped delivery object from external forces. When the holding portion is exposed from the cylindrical body, the holding portion can be configured to be developed in a planar shape with its restorability. As one aspect, the holding portion is molded in a state of being curved along the circumferential direction of the main body, so that the sheet-shaped delivery object can be also configured to be protected from external forces without being accommodated in the cylindrical body.

The shaft portion and the holding portion of the main body can be made of separate materials, or can be made of the same material. When the main body is made of the same material, for example, the main body can be made of a flexible material (synthetic resin or the like). In this case, the distal end side of the main body can be molded as a flexible holding portion having a planar surface that holds the sheet-shaped delivery object, and the proximal end side of the main body can be molded as a shaft portion having rigidity by being rolled into a cylindrical shape or a substantially tubular shape. The flexible distal end side is curved and molded along the circumferential direction of the main body to protect the sheet-shaped delivery object from external forces, and the holding portion is pressed against a flat surface to be deformed into a planar surface, so that the sheet-shaped delivery object can be appropriately placed and moved. When a rigid material (for example, metal) is used on the distal end side of the main body and the distal end side of the body is curved and molded along the circumferential direction, the sheet-shaped delivery object can be more firmly protected from external forces.

It is preferable that the holding portion can be bent with respect to an axial direction of the shaft portion like a paddle (turner, spatula) in order to scooped up the sheet-shaped delivery object on a flat surface such as a petri dish or a workbench, and to move the scooped sheet-shaped delivery object onto a target site having a flat surface. For example, when the main body is made of a rigid material (for example, metal), such an angle can be maintained by bending a boundary portion (bent portion) between the holding portion and the shaft portion at an arbitrary angle. For example, in a case where the holding portion is made of a flexible material, the holding portion can be bent at an arbitrary angle by being pressed against the flat surface, and at the same time, the holding portion can be returned to the original state parallel to the shaft portion with its restorability by being separated from the flat surface. When the case where the holding portion and the shaft portion are parallel (horizontal) is defined as 0 degrees, an arbitrary angle can be, for example, 10 to 100 degrees, preferably 45 to 90 degrees.

In one aspect, the holding portion can be configured to have a tapered portion expanding in a tapered manner from the distal end of the shaft portion, and a placement portion extending from the tapered portion. When the main body (shaft portion) is drawn in the proximal direction from the distal end of the cylindrical body, the tapered portion is pressed against an opening of the cylindrical body, and is smoothly curved without being caught by a curve of the tapered portion. As a result, it is possible to place the sheet-shaped delivery object on the holding portion with a width larger than the inner diameter of the cylindrical body and to curvedly store the sheet-shaped delivery object along the inner periphery of the cylindrical body. In addition, it is preferable that the corner on the distal end side of the holding portion is chamfered to be rounded so that the main body (holding portion) can be smoothly pushed in the distal end direction from the proximal end of the cylindrical body.

Further, in one aspect, the holding portion can be also configured to store the sheet-shaped delivery object with a width larger than the inner periphery of the cylindrical body. That is, when the width of the placement portion is configured to be larger than the inner periphery of the cylindrical body, the holding portion with a width larger than the inner periphery of the cylindrical body can be accommodated by being wound such that both end portions of the placement portion curved in the cylindrical body are in contact with each other at the upper portion of the cylindrical body and the back surfaces are pressed against each other. In one aspect, both end portions of the placement portion can be also configured to be folded back at least on the proximal end side, whereby the folded back surfaces come into contact with each other at both end portions of the placement portion curved in the cylindrical body, so that the placement portion is more smoothly wound such that the back surfaces are pressed against each other.

When winding occurs on the proximal end side of the placement portion, the distal end side of the placement portion is also wound in the winding direction on the proximal end side in cooperation, and thus the folding back of the proximal end sides of both end portions can serve as a winding core. The folding can be realized by folding both ends of the placement portion inward, winding both ends of the placement portion inward in a roll shape, or the like. It is also possible to provide strength by filling a groove on the surface side of the placement portion formed when both ends of the placement portion are folded inward. Both end portions of the placement portion may be folded back at least on the proximal end side, and the portion on which the sheet-shaped delivery object is placed can be configured as a planar surface. By configuring such that the folding angle of both end portions with respect to the placement portion increases from the distal end side to the proximal end side, the distal end side can have a function of placing the sheet-shaped delivery object, and the proximal end side can have a function as a winding core portion. In the present invention, a folded portion of both end portions of the placement portion may be referred to as a "folded portion".

In the present invention, the "sliding body" refers to a long member that slides on the holding portion. The sliding body is configured to move in parallel with the holding portion to slide the sheet-shaped delivery object on the holding portion while pressing the sheet-shaped delivery object, thereby slidably moving the sheet-shaped delivery object to the target site. The sliding body can be arranged parallel to the main body, preferably at least on a part of the main body, and further preferably parallel to the holding portion. For example, in a case where the main body and the sliding body are accommodated together in one cylindrical body or the like inserted intraperitoneally into the body cavity and delivered to the target site, one hole can be provided in the abdomen, which is minimally invasive. Then, by gripping the proximal end of the shaft portion of the main body with one hand and gripping the proximal end of the sliding body with the other hand, both the main body and the sliding body can be operated from the same direction, and thus operability is excellent.

As described above, the shaft portion of the main body is configured to be hollow, and the sliding body is inserted into the shaft portion, so that the sliding body can be also configured to be accurately positioned with respect to the main body. That is, when the sliding body is moved forward and backward in the hollow shaft portion, the sliding direction of the sliding body with respect to the main body and the radial position of the sliding body with respect to the main body can be fixed, so that the sliding body can be accurately positioned with respect to the holding portion. This is advantageous because a pressed position (a position at which the sliding body abuts on the holding portion) can be accurately set when the sheet-shaped delivery object on the holding portion is slid while being pressed by the sliding body. The positioning of the sliding body with respect to the main body can also be realized by accommodating both the sliding body and the main body (having a solid or hollow shaft portion) in the cylindrical body so as to be movable forward and backward.

In the present invention, the "hollow" refers to a cylindrical shape, a substantially tubular shape, or the like, and the substantially tubular shape also includes a shape in which, when both ends of a strip-shaped material are curved along the circumferential direction of the long axis, both ends are not connected to each other (a shape in which a longitudinal slit is formed in a cylindrical body). By configuring the hollow shaft portion so that an endoscope or the like can be inserted in addition to the sliding body, it is possible to observe whether the sheet-shaped delivery object is appropriately pressed by the sliding body, accurately moved to the target site, and the like. The outer diameter of the shaft portion is not particularly limited, but can be, for example, 2 to 24 mm, preferably 4 to 18 mm. The inner diameter of the shaft portion is not particularly limited, but can be, for example, 1 to 22 mm, preferably 2 to 16 mm.

In one aspect, the distal end portion of the sliding body can be configured as a pressing portion having a three-dimensional shape or a planar shape for pressing the sheet-shaped delivery object. The three-dimensional shape refers to a shape including at least one point (vertex) in contact with the sheet-shaped delivery object, and includes, for example, a sphere, an ellipsoid, a polyhedron (tetrahedron, hexahedron, octahedron, or the like), and the like. The planar shape refers to a plate shape with a planar surface covering the surface of the sheet-shaped delivery object, and can be, for example, a disk shape when the sheet-shaped delivery object is circular and a square plate shape when the sheet-shaped delivery object is quadrangular. In a case where the sheet-shaped delivery object on the holding portion is pressed by the pressing portion having a three-dimensional shape, since the vertex of the pressing portion acts as a force point on the sheet-shaped delivery object, the sheet-shaped delivery object can be easily slidably moved while minimizing friction with the holding portion.

In addition, when the sheet-shaped delivery object on the holding portion is pressed by the pressing portion having a planar shape, even if the sheet-shaped delivery object is damaged or deformed, the sheet-shaped delivery object can be formed into an appropriate shape by being covered with a surface and pressed, and slidably moved. The material of the three-dimensional pressing portion is preferably made of an elastic body so that an excessive force is not applied to the sheet-shaped delivery object. The shape of the pressing portion is preferably a three-dimensional shape in which a vertex pressing the sheet-shaped delivery object is rounded (sphere, ellipsoid, or the like), and is preferably a three-dimensional shape in which both the distal end side and the proximal end side are chamfered (sphere, ellipsoid) from the viewpoint of taking in and out the sheet-shaped delivery object from the shaft portion or the cylindrical body. In one embodiment, a discharge port through which fibrin or the like can be discharged on the main body or the sliding body is provided, the sheet-shaped delivery object on the holding portion is reinforced with fibrin, and physiological saline is allowed to flow from the discharge port, so that wetting of the sheet-shaped delivery object and slippage with the holding portion can be also configured to be maintained and improved.

As described above, when the holding portion is configured to be bendable at an arbitrary angle with respect to the axial direction of the shaft portion, the sliding body is preferably configured to be deflected in accordance with the bending of the holding portion. For example, when the holding portion is pressed against the target site and bent with respect to the shaft portion, the sliding body sliding parallel to the shaft portion abuts on the bent holding portion. At this time, the sliding body is deflected in accordance with the bending curve of the holding portion, so that the sheet-shaped delivery object on the holding portion can be slidably moved horizontally onto the target site while being pressed. The sliding body can realize a structure deflected by, for example, inserting a metal coil therein, but those skilled in the art can easily realize the sliding body by appropriately adopting various materials and structures.

Materials of the main body and the sliding body are not particularly limited, and various known materials can be used alone or in combination. Examples of such a material include a metal, a soft vinyl chloride resin, a polyurethane resin, a polyurethane elastomer, a silicone rubber, a natural rubber, a synthetic rubber, a styrene-ethylene-butylene-styrene copolymer (SEBS), a styrene-ethylene-propylene-styrene copolymer (SEPS), an ethylenevinyl acetate copolymer (EVA), a polyamide resin and a polyamide elastomer such as nylon, a polyester resin and a polyester elastomer such as polyethylene terephthalate (PET), an olefin-based resin such as polyethylene, a fluororubber, and a fluororesin, and one or a combination of two or more of these can be used.

In the present invention, the "slidably moved" refers that the sheet-shaped delivery object is laterally slipped on the holding portion by advancing the sliding body toward the target site while pressing the sheet-shaped delivery object on the holding portion by the sliding body. In general, the sheet-shaped delivery object is often pinched with tweezers or the like and then lifted and moved, but at this time, the sheet-shaped delivery object may be broken. On the other hand, the medical device of the present invention can minimize breakage of the sheet-shaped delivery object by utilizing a simple mechanism of slidably moving the sheet-shaped delivery object by the sliding body. Thus, it is preferred that at least the portion of the holding portion on which the sheet-shaped delivery object is placed be low frictional so that such lateral slip suitably occurs. In the present invention, the sheet-shaped delivery object can be pressed to the distal end of the holding portion, and further, can be continuously pressed by one operation when the sheet-shaped delivery object is applied from the holding portion to the target site.

In the present invention, the "low frictional" means that the frictional properties between the sheet-shaped delivery object and the holding portion is low. The low friction part of the holding portion can be realized by being made of a low friction material or applying a low friction material to the surface (hydrophilic coating, fluorine coating, or the like). Those skilled in the art can use a variety of materials to investigate the frictional properties between the sheet-shaped delivery object and the holding portion to find an optimal low friction material.

In the present invention, the "liquid" is composed of at least one component, and the component is not particularly limited, but is composed of, for example, a liquid such as water, an aqueous solution, a non-aqueous solution, a suspension, or an emulsion. A component constituting the liquid is not particularly limited as long as it has little influence on the sheet-shaped delivery object. In a case where the sheet-shaped delivery object is a membrane made of a biological material, the component constituting the liquid is preferably a biocompatible component, that is, a component that does not cause an undesirable action such as an inflammatory reaction, an immune reaction, or an intoxication reaction on a biological tissue or a cell, or at least has a small action.

In the present invention, the "cylindrical body" refers to a pipe-shaped (hollow) long object having a space inside. The cylindrical body has openings at both ends thereof, and the main body on which the sheet-shaped delivery object is placed can be moved to the target site on the distal end side through the internal space. The cylindrical body is not particularly limited as long as it has rigidity enough to protect the sheet-shaped delivery object, the main body, the sliding body, and the like from external forces, and any cylindrical body including a commercially available cylindrical body (lumen tube or the like) can be used. Examples of a material of the cylindrical body include, but are not limited to, polyethylene, polypropylene, fluororesin, polyethylene terephthalate, polymethyl methacrylate, polyamide resin, polystyrene, polycarbonate, polyimide, polyetherimide, polyetheretherketone, glass, a metal (for example, iron, stainless steel, aluminum, copper, or brass).

The cylindrical body may be made of a flexible material, and in this case, even if a passage to be inserted into the body is curved in laparoscopic surgery, thoracoscopic surgery, or the like, the cylindrical body can be curved in accordance with a form of the passage, or can be bent in order to adjust a relative angle between the cylindrical body and the target site. By configuring the cylindrical body so that an endoscope or the like can be inserted in addition to the main body and/or the sliding body, it is possible to observe whether the sheet-shaped delivery object is appropriately curved in the cylindrical body, wound, appropriately pressed by the sliding body, accurately moved to the target site, and the like. The medical device of the present invention may further include such a cylindrical body. The outer diameter of the cylindrical body is not particularly limited, but can be, for example, 4 to 26 mm, preferably 6 to 20 mm. The inner diameter of the cylindrical body is not particularly limited, but can be, for example, 3 to 25 mm, preferably 5 to 19 mm.

Hereinafter, a medical device according to a preferred embodiment of the present invention will be described in detail with reference to the drawings.

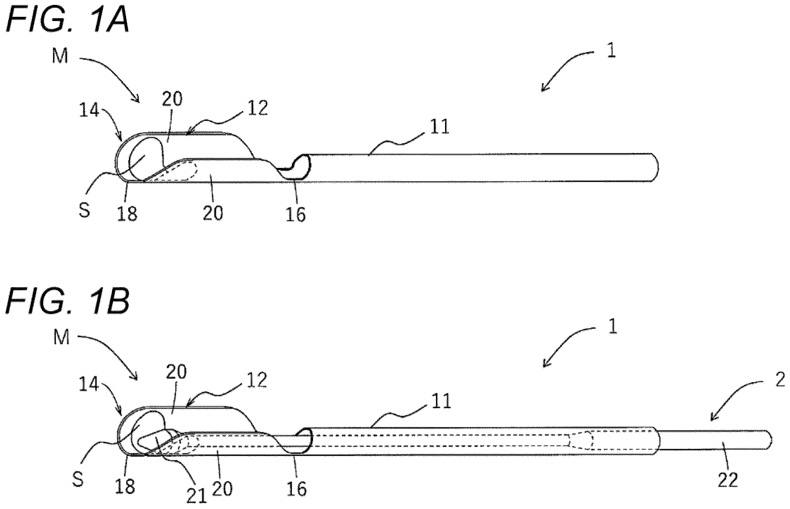

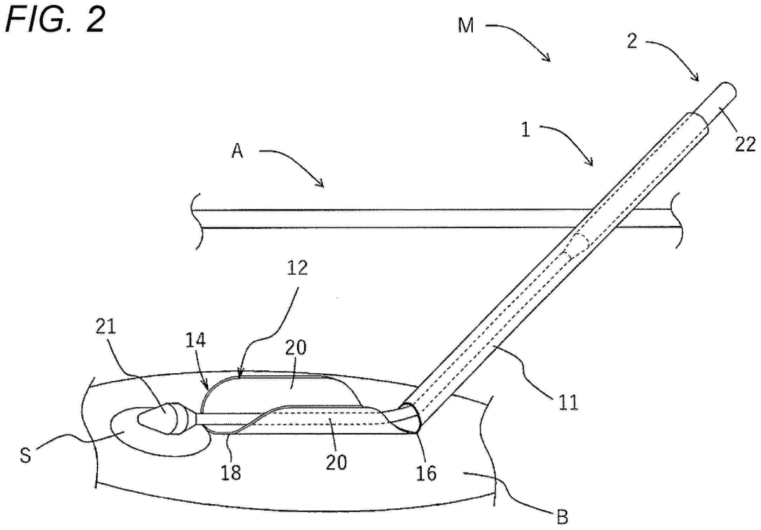

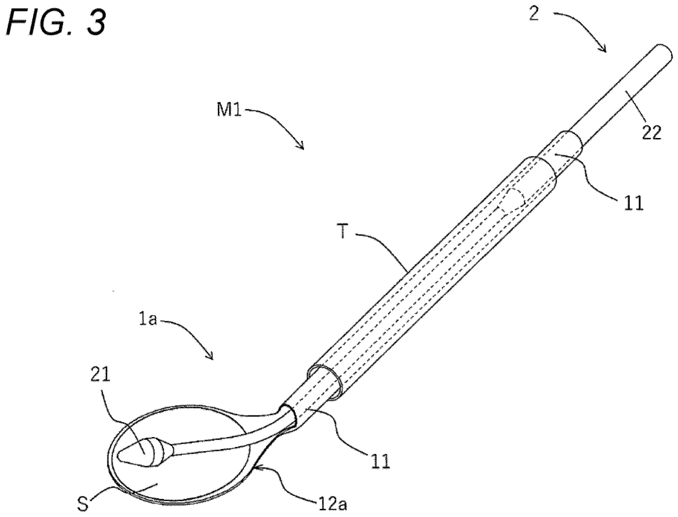

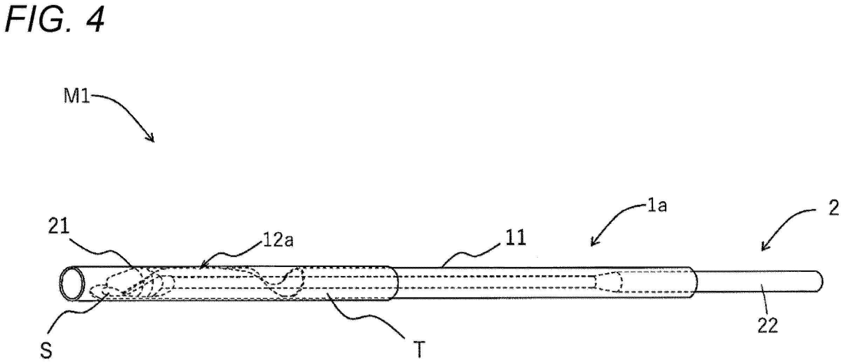

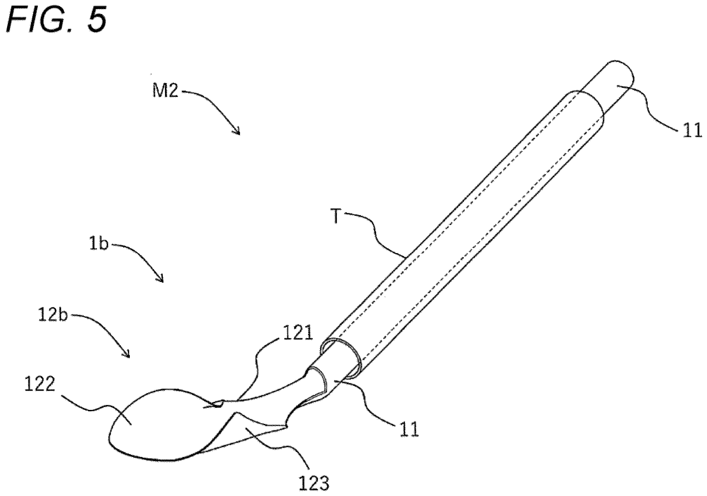

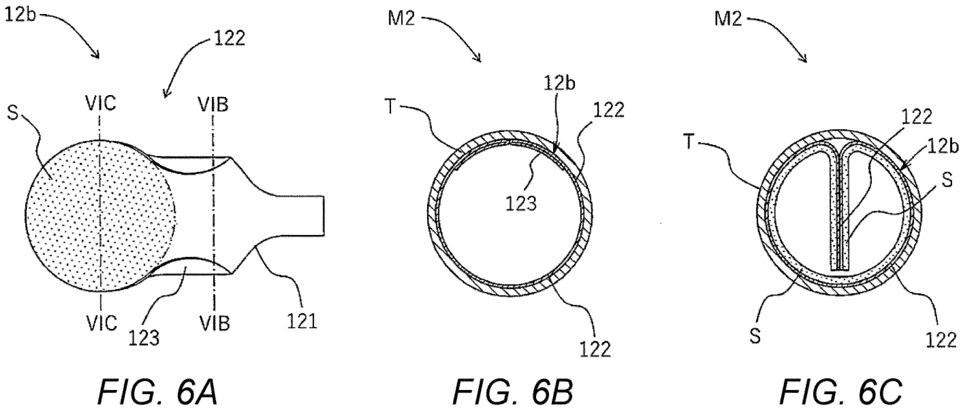

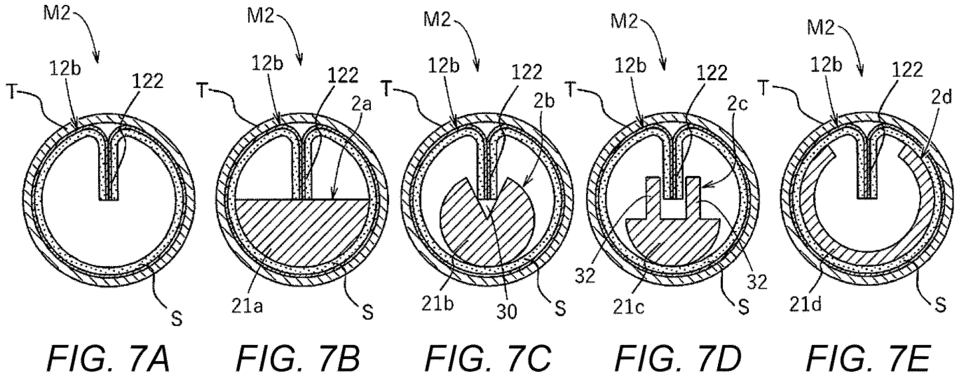

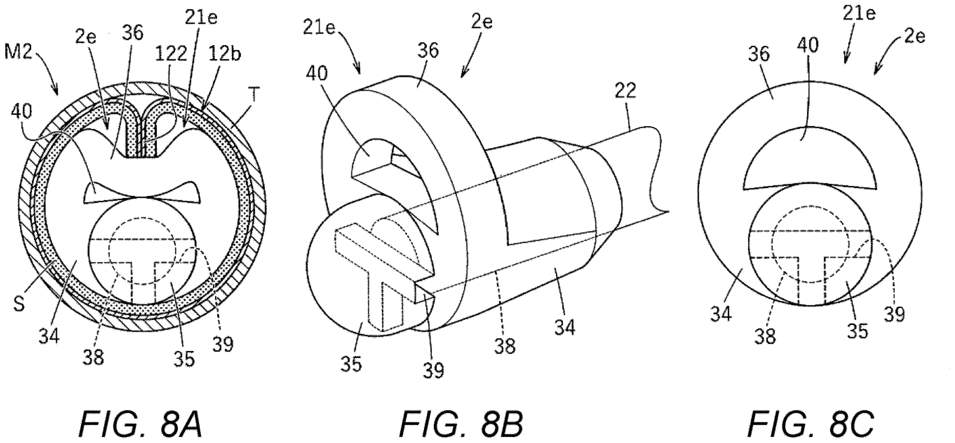

Fig. 1 is a conceptual diagram illustrating a medical device according to a first embodiment of the present invention. Fig. 2 is a diagram illustrating a usage example of the medical device according to the first embodiment of the present invention. Fig. 3 is a conceptual diagram illustrating a medical device according to a second embodiment of the present invention. Fig. 4 is a diagram illustrating a usage example of the medical device according to the second embodiment of the present invention. Fig. 5 is a conceptual diagram illustrating a medical device according to a third embodiment of the present invention. Figs. 6A to 6C are diagrams illustrating a usage example of the medical device according to the third embodiment of the present invention. Figs. 7A to 7E and Figs. 8A to 8C are diagrams illustrating a modification of the medical device according to the third embodiment of the present invention.

In the drawings in the present application, the size of each member is appropriately emphasized for ease of description, and each member illustrated does not indicate an actual size.

### (First Embodiment)

First, a first embodiment of the present invention will be described. As illustrated in Fig. 1A, a medical device M according to a first embodiment of the present invention includes a main body 1 having a long shaft portion 11 and a holding portion 12 provided at a distal end of the shaft portion 11 for holding a sheet-shaped delivery object S. The main body 1 is made of a plastic material such as metal.

The shaft portion 11 constituting a proximal end side of the main body 1 is hollow molded in a rounded shape. That is, the shaft portion 11 is formed in a cylindrical shape. The shaft portion 11 is formed in a size and a shape that can be easily gripped by a user. The shaft portion 11 functions as a handle (grip) portion.

The holding portion 12 constituting a distal end side of the main body 1 is molded in a state of being curved along a circumferential direction of the main body 1. The holding portion 12 functions as a curved spatula portion that protects the sheet-shaped delivery object S from external forces. The holding portion 12 has a placement portion 14 that instructs the sheet-shaped delivery object S, and a connection portion 16 that connects the placement portion 14 and the shaft portion 11.

The placement portion 14 is formed in a U shape when viewed from a distal end direction. The placement portion 14 includes a central support portion 18 curved in a semicircular shape along the circumferential direction of the shaft portion 11 and a pair of side support portions 20 provided at both end portions of the central support portion 18 in a width direction. A proximal end portion of the central support portion 18 is connected to the connection portion 16.

The side support portion 20 protrudes in a direction in which the inner surface of the central support portion 18 faces. The pair of side support portions 20 is disposed so as to face each other. The inner surface of the placement portion 14 forms a placement surface on which the sheet-shaped delivery object S is placed. The placement surface is curved in a U shape when viewed from the distal end direction.

The connection portion 16 is formed in an arc shape when viewed from the distal end direction. The inner surface of the connection portion 16 connects the inner surface of the shaft portion 11 and the inner surface (placement surface) of the placement portion 14 without a step. The connection portion 16 is bendable so that the direction of the placement portion 14 with respect to the shaft portion 11 can be changed (see Fig. 2).

As illustrated in Fig 1B, the medical device M can optionally and selectively include a long sliding body 2 that slides on the holding portion 12 parallel to the main body 1. The sliding body 2 has a long sliding shaft 22 and a pressing portion 21 provided at a distal end portion of the sliding shaft 22.

The pressing portion 21 may have an ellipsoid shape, and can press the sheet-shaped delivery object S on the holding portion 12 by a vertex of the pressing portion 21 to prevent sliding down of the sheet-shaped delivery object S from the holding portion 12. The sliding body 2 can be accommodated in the hollow shaft portion 11 so as to be movable forward and backward. Then, by advancing the pressing portion 21 from the shaft portion 11, it is possible to slidably move the sheet-shaped delivery object S in the distal end direction while pressing the sheet-shaped delivery object S. Optionally, by retracting the pressing portion 21 into the shaft portion 11, the sheet-shaped delivery object S can be drawn into the shaft portion 11 to be accommodated.

The sliding body 2 that moves forward and backward in the shaft portion 11 of the main body 1 can fix the sliding direction with respect to the main body 1 and the radial position with respect to the main body 1, so that the pressing portion 21 can be accurately positioned with respect to the holding portion 12 (and the sheet-shaped delivery object S thereon). Since the pressing portion 21 has a dome-shaped ellipsoid shape on both the distal end side and the proximal end side, the pressing portion 21 can be smoothly moved without being caught by the shaft portion 11 or the holding portion 12 when advancing from the shaft portion 11, retracting into the shaft portion 11, pulling back from the target site onto the holding portion 12, and the like. In addition, since a vertex of the ellipsoidal shape in contact with the pressing portion 21 is rounded, an excessive force is less likely to be applied to the sheet-shaped delivery object S. The pressing portion 21 can also be formed of an elastic body so as not to apply excessive force.

As illustrated in Fig. 2, when the medical device M is used, the sheet-shaped delivery object S is placed on the holding portion 12, and for example, an abdomen A is incised, and then the medical device M is inserted intraperitoneally into the body cavity and delivered to a target site B. At this time, the sheet-shaped delivery object S is protected inside the curved holding portion 12, so that problems such as detachment of the sheet-shaped delivery object S due to contact with other portions at the time of insertion can be minimized. Also, in order to prevent sliding down of the sheet-shaped delivery object S, a thread guide can be provided between the sheet-shaped delivery object S and the target site B before the sheet-shaped delivery object S is inserted into the body cavity. By pressing the sheet-shaped delivery object S onto the holding portion 12 by the pressing portion 21, sliding down can also be prevented. The sheet-shaped delivery object S can also be inserted into the body cavity in a state of being drawn into the shaft portion 11 by the pressing portion 21.

As illustrated in Fig. 2, the holding portion 12 can be pressed against the target site B and bent at an arbitrary angle with respect to an axial direction of the shaft portion 11, due to its plasticity. As a result, the holding portion 12 and the target site B can be made horizontal. Furthermore, by configuring the sliding body 2 to be deflected in accordance with the curve of bending of the holding portion 12, the bent holding portion 12 and the pressing portion 21 of the deflected sliding body 2 can be kept horizontal (positioned on the same plane). As a result, by advancing the sliding body 2 while pressing the sheet-shaped delivery object S on the holding portion 12 by the pressing portion 21, it is possible to slide horizontally onto the target site B. Even when the pressing portion 21 on the target site B is pulled back onto the holding portion 12, the dome-shaped proximal end side of the pressing portion 21 is not caught by the holding portion 12.

Similarly, even when the sheet-shaped delivery object S on the flat surface such as a petri dish is scooped up (placed) on the holding portion 12, the placement portion 14 is pressed against the flat surface and the connection portion 16 is bent, whereby the placement portion 14 is made horizontal with respect to the flat surface, and the sheet-shaped delivery object S can be easily moved onto the placement portion 14. By molding the holding portion 12 using a rigid material, it is also possible to configure so that the curved shape of the holding portion 12 and the bending angle with respect to the shaft portion 11 are maintained. The shaft portion 11 is not necessarily hollow, and can be molded solid. Optionally and selectively, both the solid or hollow shaft portion 11 and the sliding body 2 are stored in a cylindrical body (not shown), so that the sliding direction of the sliding body 2 with respect to the main body 1 and the radial position with respect to the main body 1 can also be fixed. The holding portion 12 can also be molded in a curved shape using a flexible material.

As described above, the present invention can efficiently apply the sheet-shaped delivery object to a target site while maintaining the shape of the sheet-shaped delivery object by a simple mechanism and a simple operation, and therefore has a large advantage in terms of operability and manufacturing cost.

### (Second Embodiment)

Hereinafter, a medical device M1 according to a preferred second embodiment of the present invention will be described in detail with reference to the drawings.

As illustrated in Fig. 3, a medical device M1 according to a second embodiment of the present invention includes a main body 1a having a long shaft portion 11 and a holding portion 12a provided at a distal end of the shaft portion 11 for holding a sheet-shaped delivery object S. In the present embodiment, a description will be given on the assumption that the main body 1a is made of a flexible material, a distal end side (holding portion 12a) is molded as a planar surface, and the main body 1a is used to be accommodated in a cylindrical body T so as to be movable forward and backward.

The main body 1a is dimensioned so that the main body 1a itself can also be accommodated inside the cylindrical body T so as to be movable forward and backward in a state where the sliding body 2 is accommodated inside the shaft portion 11. As a result, for example, the main body 1a and the sliding body 2 can be inserted together into one cylindrical body (tubular body having a lumen (tube)) inserted intraperitoneally into the body cavity and delivered to a target site (not illustrated), which is minimally invasive. Also, as a result, the main body 1a and the sliding body 2 can be operated by gripping the proximal end of the shaft portion 11 with one hand and gripping the proximal end of the sliding body 2 with the other hand from the same direction outside the body while maintaining the relative positional relationship between the main body 1a and the sliding body 2. By dimensioning the main body 1a so that an endoscope can be inserted into the shaft portion 11 or the cylindrical body T, the endoscope can be further operated from the same direction.

The width of the distal end side (placement portion) of the holding portion 12a on which the sheet-shaped delivery object S is placed is configured to be larger than the inner diameter of the cylindrical body T and smaller than the inner periphery of the cylindrical body T. As a result, the holding portion 12a (and the sheet-shaped delivery object S thereon) can be stored along the inner periphery of the cylindrical body T in a curved state. The proximal end side of the holding portion 12a is configured to widen in a tapered manner from the distal end of the shaft portion 11 so that the holding portion 12a is smoothly curved. As illustrated in the drawing, the holding portion 12a can be bent with respect to the axial direction of the shaft portion 11 by being pressed against the flat surface, and the holding portion 12a can be made horizontal with respect to the flat surface.

As illustrated in Fig. 4, when the medical device M1 is used, the sheet-shaped delivery object S is placed on the holding portion 12a, and the pressing portion 21 is advanced to press the sheet-shaped delivery object S on the holding portion 12a. Then, the main body 1a is drawn inward from the distal end of the cylindrical body T, and the tapered proximal end side of the holding portion 12a is pressed against a distal end opening of the cylindrical body T to be curved. When the main body 1a is further drawn, the curvature of the distal end side on which the sheet-shaped delivery object S is placed starts in cooperation with the curvature of the proximal end side of the holding portion 12a. Since the holding portion 12a is curved along the inner periphery of the cylindrical body T, the sheet-shaped delivery object S with a width larger than the inner diameter of the cylindrical body T and smaller than the inner periphery of the cylindrical body T can be accommodated.

The sheet-shaped delivery object S is pressed onto the holding portion 12a by the pressing portion 21 of the sliding body 2, is protected inside the curved holding portion 12a, and is further doubly protected inside the cylindrical body T. By being inserted into the body cavity intraperitoneally in this state, it is possible to deliver the sheet-shaped delivery object S to the target site while simultaneously achieving double protection and slide-down prevention of the sheet-shaped delivery object S. Then, as illustrated in Fig. 3, the main body 1a can be inserted into the cylindrical body T and advanced from the distal end, and the holding portion 12a can be developed on a planar surface with its restorability. For example, the holding portion 12a developed on the planar surface can be entered under the sheet-shaped delivery object S floating in a liquid in a petri dish and scooped up like a spatula. As in the first embodiment, the holding portion 12a may be curved and molded along the circumferential direction of the main body 1. The pressing portion 21 may be used to protrude from the distal end of the holding portion 12a, hook the sheet-shaped delivery object S in the petri dish, and then draw the sheet-shaped delivery object S onto the holding portion 12a.

As described above, the present invention can efficiently apply the sheet-shaped delivery object to a target site while maintaining the shape of the sheet-shaped delivery object by a simple mechanism and a simple operation, and therefore has a large advantage in terms of operability and manufacturing cost.

### (Third Embodiment)

Hereinafter, a medical device M2 according to a preferred third embodiment of the present invention will be described in detail with reference to the drawings.

As illustrated in Fig. 5, a medical device M2 according to a third embodiment of the present invention includes a main body 1b having a long shaft portion 11 and a holding portion 12b provided at a distal end of the shaft portion 11 for holding a sheet-shaped delivery object S. The holding portion 12b has a tapered portion 121 widening in a tapered manner from a distal end of the shaft portion 11 and a placement portion 122 extending from the tapered portion 121. In the present embodiment, the width of the distal end side of the holding portion 12b on which the sheet-shaped delivery object S is placed (placement portion 122) is configured to be larger than the inner periphery of the cylindrical body T, and both end portions of the placement portion 122 in the width direction are folded inward on the proximal end side (folded portion 123).

As illustrated in Fig. 6A, the holding portion 12b is configured such that the proximal end side is the tapered portion 121 and the distal end side is the placement portion 122, the distal end side of the placement portion 122 is configured as a planar surface on which the sheet-shaped delivery object S can be placed, and both end portions of the proximal end side of the placement portion 122 are folded inward (folded portion 123). As one aspect, the most proximal end sides of both end portions are folded back at 180 degrees with respect to the placement portion 122 (so that back surfaces of both end portions are horizontal to the surface), and the most distal end sides of both end portions are not folded back with respect to the placement portion 122 (0 degrees). That is, the folding angle of both end portions with respect to the placement portion 122 can be configured to increase from the distal end side to the proximal end side of the placement portion 122.

Fig. 6B shows a cross-sectional view taken along line VIB-VIB, and Fig. 6C shows a cross-sectional view taken along line VIC-VIC when the placement portion 122 of Fig. 6A is accommodated in the cylindrical body T. As illustrated in Fig. 6B, when the placement portion 122 is drawn into the cylindrical body T, first, the proximal end side of the placement portion 122 is curved in cooperation with the curvature of the tapered portion 121, and both end portions of the curved folded portion 123 are in contact with each other at the upper portion of the cylindrical body T. At this time, since both end portions are folded back, the folded back surfaces are pressed against each other. Then, when the placement portion 122 is further drawn into the cylindrical body T, as illustrated in Fig. 6C, the back surfaces of the placement portion 122 are wound so as to be pressed against each other.

As described above, when winding occurs on the proximal end side of the placement portion 122, the distal end side which is not folded back is also wound in the winding direction in cooperation, and thus the folded portion 123 can serve as a winding core for winding the placement portion 122. On the distal end side without folding back at both end portions, the sheet-shaped delivery object S can be placed up to both ends, and the sheet-shaped delivery object S is wound while the back surfaces are pressed against each other and extended vertically downward (Fig. 6C), so that the sheet-shaped delivery object S with a width larger than the inner periphery of the cylindrical body T can be accommodated. As illustrated in Fig. 6C, when the sheet-shaped delivery object S is extended from the upper portion to the lower portion of the cylindrical body T, the sheet-shaped delivery object S with a diameter up to 1.6 times the inner periphery of the cylindrical body T as an upper limit can be accommodated. The folded portion 123 can be also prepared as a separate member and attached to or fixed to the holding portion 12b by adhesion or welding.

As illustrated in Fig. 7A, the sheet-shaped delivery object S may be extended to the center of the cylindrical body T. As illustrated in Fig. 7B, a pressing portion 21a of a sliding body 2a may be accommodated in the lower space of the sheet-shaped delivery object S inside the cylindrical body T. The pressing portion 21a has a semicircular cross section. The medical device M2 according to the present embodiment may have sliding bodies 2b to 2e according to first to fourth modifications instead of the sliding body 2a.

As illustrated in Fig. 7C, the pressing portion 21b of the sliding body 2b according to the first modification has a circular cross section, and a groove 30 is formed on the upper side. An end portion of the sheet-shaped delivery object S is inserted into the groove 30 in a state where the sheet-shaped delivery object S is stored in the cylindrical body T.

As illustrated in Fig. 7D, the pressing portion 21c of the sliding body 2c according to the second modification has a semicircular cross section, and two ridges 32 are provided in a protruding manner with a space therebetween. An end portion of the sheet-shaped delivery object S is inserted between the two ridges 32 in a state where the sheet-shaped delivery object S is stored in the cylindrical body T.

As illustrated in Fig. 7E, the pressing portion 21d of the sliding body 2d according to the third modification has a form conforming to the curvature of the placement portion 122, and has a structure that does not obstruct the folded placement portion 122.

As illustrated in Figs. 8A to 8C, a pressing portion 21e of the sliding body 2e according to the fourth modification includes a pressing main body 34 for pressing the sheet-shaped delivery object S and a grip portion 36 provided on the pressing main body 34. The pressing main body 34 extends in the distal end direction from the distal end of the sliding shaft 22. A distal end surface 35 of the pressing main body 34 is formed in a hemispherical shape. In the pressing main body 34, a first hole 38 and a second hole 39 for priming communicating with a lumen of the hollow sliding shaft 22 are formed.

The first hole 38 extends from the proximal end to the distal end side of the pressing main body 34. The first hole 38 communicates with the lumen of the sliding shaft 22. The second hole 39 communicates with the distal end of the first hole 38. The second hole 39 is formed in a T shape. The second hole 39 opens in a plurality of directions (for example, three directions) on an outer surface of a distal end portion of the pressing main body 34. Specifically, for example, the second hole 39 opens toward both sides of the pressing main body 34 and opens downward (sheet-shaped delivery object S).

The grip portion 36 protrudes upward in an arch shape from an intermediate portion in the extending direction of the pressing main body 34. The grip portion 36 extends in an arc shape (semicircular shape). A hole 40 opened on both sides in an extending direction of the pressing main body 34 is provided between the grip portion 36 and the pressing main body 34. Thus, the grip portion 36 can be gripped by tweezers (not illustrated) or the like to move the pressing portion 21e with respect to the holding portion 12b. The grip portion 36 is made of a soft resin material. As illustrated in Fig. 8A, the grip portion 36 is a flexible protrusion that is deformed by contact of the folded placement portion 122.

When the sliding body 2e having such a pressing portion 21e is provided, the sheet-shaped delivery object S is placed on the holding portion 12b from a petri dish, and then the pressing portion 21e can be easily disposed on the sheet-shaped delivery object S by picking the grip portion 36 with tweezers or the like. As a result, the sheet-shaped delivery object S can be held by the holding portion 12b by the pressing portion 21e.

In these modifications, the sheet-shaped delivery object S is not necessarily pressed by the pressing portions 21a to 21e in the cylindrical body T. For example, the pressing portions 21a to 21e may be retracted onto the tapered portion 121 when the holding portion 12b is in the cylindrical body T, and may be moved onto the holding portion 12b when the sheet-shaped delivery object S is slidably moved. Further, since the sheet-shaped delivery object S wound inside the placement portion 122 is less likely to slide down, the lower space is not an essential configuration.

The folding angle of both end portions is not particularly limited as long as the back surfaces are in contact with each other at the upper portion of the cylindrical body T, and can be, for example, 60 to 180 degrees, preferably 90 to 180 degrees when the case of parallel (horizontal) to the placement portion 122 is defined as 0 degrees. A person skilled in the art can appropriately set the folding angle of both end portions according to the inner diameter and inner periphery of the cylindrical body T, the width of the placement portion 122, and the like. The folding includes folding back in a roll shape, making inside of the folded portion solid (ridge shape), and the like. As in the first embodiment, the holding portion 12b may be curved and molded along the circumferential direction of the main body 1. In the present embodiment, the medical device M only needs to include at least the main body 1b, and may optionally and selectively further include the sliding bodies 2a to 2e and/or the cylindrical body T. The shaft portion 11 may be configured to be either solid or hollow.

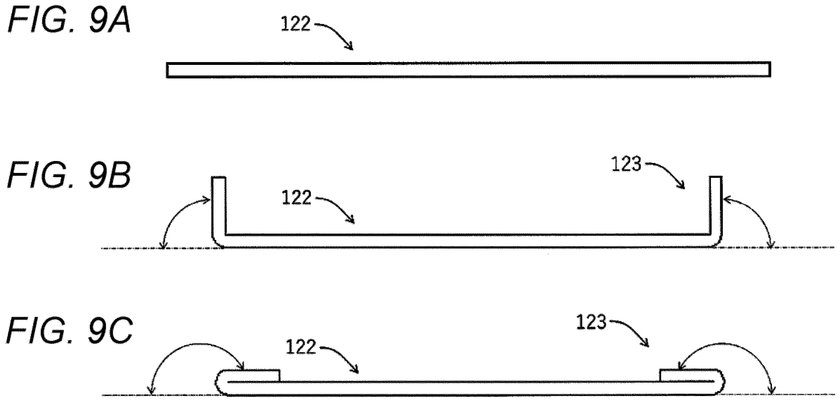

Figs. 9A to 9C are conceptual diagrams of the folded portion 123. As illustrated in Figs. 9A to 9C, the folding angle refers to an angle formed by the folded portion 123 and the placement portion 122. As illustrated in Fig. 9A, when the placement portion 122 is not folded back, the angle formed by the folded portion 123 and the placement portion 122 is 0 degrees. As illustrated in Fig. 9B, when the folded portion 123 is folded back perpendicularly to the placement portion 122, the angle formed by the folded portion 123 and the placement portion 122 is 90 degrees. As illustrated in Fig. 9C, when the folded portion 123 is horizontally folded back with respect to the placement portion 122, the angle formed by the folded portion 123 and the placement portion 122 is 180 degrees. The folding angle may be referred to as an outer angle of the folded portion 123.

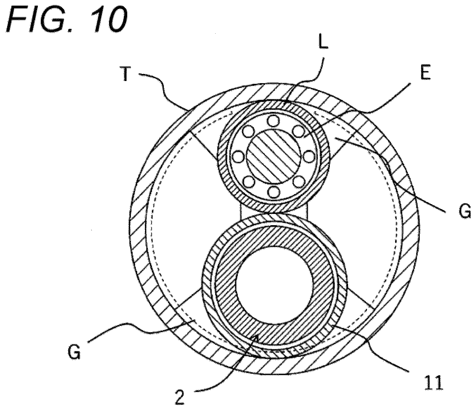

Fig. 10 shows a conceptual diagram in a case where the shaft portion 11, the sliding body 2, and an endoscope E are inserted into the cylindrical body T. As illustrated in Fig. 10, a lumen L of the endoscope E and the shaft portion 11 are each fixed to the upper side and the lower side in the cylindrical body T with an adhesive G or the like, and can be configured such that forward and backward in the lumen L of the endoscope E and forward and backward in the shaft portion 11 of the sliding body 2 independently occur without interference.

As described above, the present invention can efficiently apply the sheet-shaped delivery object to a target site while maintaining the shape of the sheet-shaped delivery object by a simple mechanism and a simple operation, and therefore has a large advantage in terms of operability and manufacturing cost.

Although the medical device according to the present invention has been described above based on the preferred embodiment, the present invention is not limited thereto, and various modifications can be made. For example, a hub with luer taper to which a syringe or the like can be connected can be provided at a proximal end portion of the hollow sliding body. In addition, an opening for moistening the sheet-shaped delivery object can be provided on the distal end side of the shaft portion (hollow) from the pressing portion. Further, a valve body capable of fixing the position of the sliding body can be provided in a gap between the sliding body and the cylindrical body to prevent the sliding body from advancing only by inclining the main body. Furthermore, the vertex of the pressing portion presses a position from the center to the front of the sheet-shaped delivery object, so that it is possible to suppress the sheet from being deflected, wrinkled, or damaged during sheet transfer.

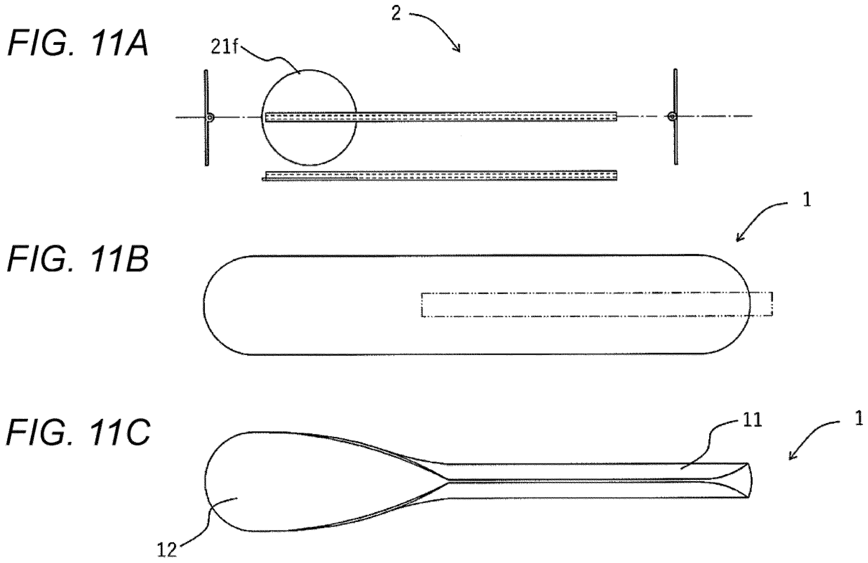

Figs. 11A to 11C are conceptual diagrams illustrating modifications of the medical device of the present invention. As illustrated in Fig. 11A, the sliding body 2 may have a disk-shaped pressing portion 21f. As illustrated in Fig. 11B, the main body 1 can be molded in a strip shape using a flexible material. Then, when the main body 1 is used, as illustrated in Fig. 11C, the proximal end side is rounded into a tubular shape with a finger or the like, so that the main body 1 can have rigidity as the shaft portion 11. Then, the distal end side is naturally curved by rounding the proximal end side, and can function as the flexible curved holding portion 12.

The use of the medical device of the present invention can be sequentially performed by the following steps.
(1) Provide a medical device
(2) Place a sheet-shaped delivery object on a holding portion
(3) Deliver a main body to a target site
(4) Apply the sheet-shaped delivery object to the target site

According to the steps (1) to (4), the sheet-shaped delivery object can be applied to the target site. In (2), a holding portion may be pressed against a petri dish containing the sheet-shaped delivery object to be bent horizontally with respect to the petri dish and/or deformed to a planar surface with respect to the petri dish so that the sheet-shaped delivery object can be easily placed on the holding portion. In (3), the sheet-shaped delivery object on the holding portion may be pressed by a sliding body to prevent sliding down. In (3), the main body and/or the sliding body may be inserted into a cylindrical body inserted intraperitoneally into the body cavity and delivered to the target site.

In (3), the holding portion may be curved in the cylindrical body to accommodate the sheet-shaped delivery object with a width larger than the inner diameter of the cylindrical body. In (3), the placement portion may be wound in the cylindrical body to accommodate the sheet-shaped delivery object with a width larger than the inner periphery of the cylindrical body. In (4), a holding portion may be pressed against the target site to be bent horizontally with respect to the target site and/or deformed to a planar surface with respect to the target site so that the sheet-shaped delivery object can be easily applied to the target site.

In (4), the sheet-shaped delivery object on the holding portion may be slidably moved by the sliding body. In (4), the sheet-shaped delivery object on the holding portion may be moved with tweezers, forceps, or the like. In (3) and/or (4), an endoscope can be inserted into the shaft portion and/or the cylindrical body to observe whether the sheet-shaped delivery object is appropriately pressed by the sliding body, appropriately curved or wound in the cylindrical body, appropriately slidably moved to the target site, and the like.

Although the present invention has been described above with reference to the illustrated embodiment, the present invention is not limited thereto. The configurations of the first to third embodiments can be appropriately combined.

In the present invention, each configuration can be replaced with any configuration that can exhibit similar functions, or any configuration can be added.

### Reference Signs List

1, 1a, 1b Main body
2, 2a to 2e Sliding body
11 Shaft portion
12, 12a, 12b Holding portion
14, 122 Placement portion
21, 21a to 21f Pressing portion
121 Tapered portion
123 Folded portion
A Abdomen
B Target site
E Endoscope
G Adhesive
L Lumen
M, M1, M2 Medical device
S Sheet-shaped delivery object
T Cylindrical body

## Claims

1. A medical device for applying a sheet-shaped delivery object to a target site, the medical device comprising a main body having a long shaft portion and a holding portion provided at a distal end of the shaft portion for holding the sheet-shaped delivery object, wherein the holding portion has a tapered portion widening in a tapered manner from the distal end of the shaft portion and a placement portion extending from the tapered portion, and both end portions of the placement portion are folded back inward at least on a proximal end side.

2. The medical device according to claim 1, wherein a folding angle of both end portions with respect to the placement portion is configured to increase from a distal end side to the proximal end side.

3. The medical device according to claim 1 or 2, wherein at least the distal end side of the placement portion on which the sheet-shaped delivery object is placed is configured to be a planar surface.

4. The medical device according to any one of claims 1 to 3, wherein the folding angle of both end portions with respect to the placement portion is configured to be 60 to 180 degrees, preferably 90 to 180 degrees.

5. The medical device according to any one of claims 1 to 4, wherein the holding portion is configured to be bendable at an arbitrary angle with respect to an axial direction of the shaft portion.

6. The medical device according to any one of claims 1 to 5, wherein the shaft portion is configured to be hollow.

7. The medical device according to any one of claims 1 to 6, further comprising a long sliding body that slides on the holding portion and/or a cylindrical body.

8. A medical device for applying a sheet-shaped delivery object to a target site, the medical device comprising a main body having a long shaft portion and a holding portion provided at a distal end of the shaft portion for holding the sheet-shaped delivery object, wherein the holding portion has a tapered portion widening in a tapered manner from the distal end of the shaft portion and a placement portion extending from the tapered portion, and when the main body is accommodated in a cylindrical body that can accommodate the main body so as to be movable forward and backward, both end portions of the placement portion are configured to be wound such that back surfaces thereof are pressed against each other.

9. The medical device according to claim 8, wherein the shaft portion is configured to be hollow.

10. A method for applying a sheet-shaped delivery object to a target site, the method comprising the steps of:
providing the medical device of any one of claims 1 to 9;
placing the sheet-shaped delivery object on the holding portion;
accommodating the medical device in the cylindrical body;
delivering the medical device to the target site; and
applying the sheet-shaped delivery object to the target site.

11. The method according to claim 10, wherein the delivery to the target site is performed by inserting the medical device into the cylindrical body intraperitoneally inserted into a body cavity.

12. The method according to claim 10 or 11, wherein the sheet-shaped delivery object is intraperitoneally inserted into the body cavity and applied to the target site.
